# EUROPEAN PATENT APPLICATION

(11) **EP 4 190 910 A1**
(43) Date of publication of application: **07.06.2023**
(21) Application number: 20947567.2
(22) Date of filing: 29.07.2020
(51) Int. Cl.: C12Q 1/68, C40B 50/18

(54) **METHOD FOR LOADING NUCLEIC ACID MOLECULE ON SOLID SUPPORT**

(71) Applicant: MGI Tech Co., Ltd., Shenzhen, Guangdong 518083 (CN)
(72) Inventor: SHAFTO, Jay, Los Altos Hill, California 94022 (US); DRMANAC, Radoje, Los Altos Hill, California 94022 (US); DRMANAC, Snezana, Los Altos Hill, California 94022 (US); XU, Chongjun, San Jose, California 95134 (US); GONG, Meihua, Shenzhen, Guangdong 518083 (CN); WANG, Ping, Shenzhen, Guangdong 518083 (CN); LONG, Xiaojuan, Shenzhen, Guangdong 518083 (CN); ZHAO, Wei, Shenzhen, Guangdong 518083 (CN); LUO, Huan, Shenzhen, Guangdong 518083 (CN); JIANG, Hui, Shenzhen, Guangdong 518083 (CN); LIU, Jian, Shenzhen, Guangdong 518083 (CN)
(74) Representative: Inspicos P/S
(86) International application number: PCT/CN2020/105621
(87) International publication number: WO 2022/021163

(57) **Abstract**

Provided are a method for loading a nucleic acid molecule, for example nucleic acid nanoball (e.g., DNA nanoball (DNB)), on a solid support, and a kit for the method

## Description

### Technical Field

The present application relates to the field of nucleic acid detection. In particular, the present application relates to a method for loading a nucleic acid molecule, such as nucleic acid nanoball (e.g., DNA nanoball (DNB)) on a solid support, and a kit used for the method.

### Background Art

At present, gene chips or DNA microarrays are widely used in high-throughput sequencing. They consist of regular array slides or simply a special glass slide coated with DNA microarrays, thousands or tens of thousands nucleic acid probes are installed on an area of several square centimeters, a large amount of gene sequence related information can be provided in one test, and thus they are important tools in research of genomics and genetics.

High-throughput sequencing technologies include DNA and RNA sequencing. Generally, the DNA or RNA samples to be tested need to be constructed into a library, such as a single-stranded circular library or a linear library, and then the library is loaded directly or in a certain way (e.g., prepared into DNB) onto a gene chip for sequencing, so that hundreds of thousands to millions of DNA molecules can be sequenced in parallel at a time. Among them, loading technology is very important for the throughput and quality of sequencing. Efficient and stable loading technology can greatly improve the throughput and quality of sequencing. In addition, it is also critical whether the signal generated by the loaded DNB or DNA is sufficient. A sufficiently large signal-to-noise ratio can have a higher discrimination rate, so as to facilitate signal extraction and improve the quality of sequencing.

When using DNB as a template for sequencing or research, it is necessary to load the DNB into a corresponding vector. For example, DNB sequencing generally requires loading DNB onto a sequencing slide, and then performing on-board detection on the sequencing slide. At present, the general process comprises preparing a single-stranded circular library, performing rolling circle amplification to form DNB containing a certain copy of nucleic acid molecules, and then directly loading the DNB onto a sequencing slide, and finally performing sequencing and data collection and analysis. However, each DNB contains a variable number of copies, some may be low in copy number, some may be high in copy number, resulting in many DNBs of different sizes. And these DNBs of different sizes will be limited by the influence of Brown/thermal motion and shearing during the loading process, resulting in that a large DNB may be loaded to two adjacent sites on the slide (that is, occupying two sites), several small DNBs may be loaded at the same site on the slide at the same time, and even individual DNBs may be lost in the solution, which leads no-loading. In particular, on ultra-high-density slides, the above-mentioned phenomena are more obvious, and these phenomena reduce the utilization rate of the slides to a certain extent, resulting in a waste of data.

In the process of high-throughput sequencing or detection, for some precious samples or libraries with limited amounts (e.g., PCR-free library, stLFR library, library with large inserts, tumor, plasma, etc.), the need for accurate loading is higher, it is necessary to consider not only the utilization rate of the slide, but also whether the signal generated by each DNB is uniform and whether the signal is strong enough, especially when the laser power is very low, it needs to be easily detected. Therefore, there is a need to develop a method that can efficiently and stably load DNB onto a slide or other support tool, and the loaded DNB can generate a signal that is uniform and strong enough for detection.

### Contents of the Invention

In the present invention, unless otherwise specified, scientific and technical terms used herein have the meanings commonly understood by those skilled in the art. Moreover, the laboratory operation steps used in this paper are all routine steps widely used in the corresponding fields. Meanwhile, for a better understanding of the present invention, definitions and explanations of related terms are provided below.

### Definition of Terms

As used herein, the term "nucleic acid" can be any type of nucleic acid. For example, the nucleic acid can be deoxyribonucleic acid (DNA), ribonucleic acid (RNA), or analog of DNA or RNA made from, for example, nucleotide analog. Nucleic acids can be single-stranded, double-stranded, or contain both single- and double-stranded sequences. Nucleic acid molecules can be derived from double-stranded DNA (dsDNA) forms (e.g., genomic DNA, PCR and amplification products, etc.), or can be derived from single-stranded forms such as DNA (ssDNA) or RNA. Furthermore, nucleic acid molecules can be linear or circular. In certain embodiments, the nucleic acid molecule can be a single-stranded circularized nucleic acid molecule (e.g., single-stranded circularized DNA).

As used herein, the term "nanoball" generally refers to a macromolecule or complex having a compact (approximately) spherical shape. Typically, the inner diameter of a nanoball ranges for example between about 1 nm and about 1000 nm, such as 1-10 nm, 10-20 nm, 20-50 nm, 50-100 nm, 100-200 nm, 200-500 nm, 500-1000 nm, preferably ranges between about 50 nm and about 500 nm.

As used herein, the term "nucleic acid nanoball" generally refers to a complex or concatemer comprising multiple copies of a nucleic acid molecule. In certain typical embodiments, these nucleic acid copies may be arranged one after the other in a continuous linear chain of nucleotides. This tandem repeat structure, together with the single-stranded nature of nucleic acids (e.g., DNA), may cause the nanoball to fold. It is readily understood that each of the multiple copies of the nucleic acid molecules in the nucleic acid nanoball may contain adapter sequences with known sequences to facilitate their amplification or sequencing. The adapter sequence for each nucleic acid molecule is usually the same, but may also be different. The nucleic acid nanoball includes, but is not limited to, DNA nanoball, which is also referred to herein as DNB.

As used herein, the term "solid support" means any insoluble substrate or matrix, such as, latex bead, dextran bead, polystyrene surface, polypropylene surface, polyacrylamide gel, gold surface, glass surface, chip and silicon wafer, to which a nucleic acid can be attached. The surface of solid support can be of any desired shape including, for example, planar, spherical, or porous as appropriate for a particular application. For example, the solid support can be a flat glass surface. The solid support can be immobilized, for example, mounted inside a flow cell, to allow interaction with a reagent-containing solution. Alternatively, the solid support may also be removable to facilitate contact with a solution in different reaction vessel.

Typically, a solid support can comprise an array of sites arranged in a predetermined pattern, wherein each site can individually attach or accommodate a nucleic acid molecule (e.g., nucleic acid nanoball). When the size of the attached nucleic acid molecule (e.g., nucleic acid nanoball) exceeds the maximum size that can be accommodated by a single site or exceeds the spacing between adjacent sites in the array, the nucleic acid molecule (e.g., nucleic acid nanoball) can occupy two or more sites, which can lead to a decreased utilization of array sites and can affect sequencing efficiency and sequencing quality.

As used herein, the term "nucleic acid polymerase" has the meaning commonly understood by those skilled in the art and includes DNA polymerase and RNA polymerase. In certain preferred embodiments, the nucleic acid polymerase is a DNA polymerase.

As used herein, the term "DNA polymerase with strand displacement activity" refers to a DNA polymerase that, in the process of extending/synthesizing a nucleic acid strand downstream, when a nucleic acid double strand is present downstream, uses a newly synthesized nucleic acid single strand to displace an existing downstream nucleic acid single strand, thereby stripping the downstream nucleic acid single strand and generating a free single strand. DNA polymerases with strand displacement activity have been used in various amplification techniques, for example, strand displacement amplification, rolling circle amplification (RCA), multiple displacement amplification, loop-mediated isothermal amplification. DNA polymerases with strand displacement activity include, but are not limited to, Bst DNA polymerase, phi29 DNA polymerase, and exo-Klenow.

As used herein, the term "phi29 DNA polymerase" refers to a DNA polymerase with strand displacement activity obtained by cloning in phage phi29 of *Bacillus subtilis.* When phi29 DNA polymerase is used for rolling circle amplification, its C-terminal domain performs polymerization function, and its N-terminal domain performs 3' to 5' exonuclease proofreading and strand displacement functions. The specific application of phi29 DNA polymerase can be found in Lieberman, K.R., G.M. Cherf. Processive replication of single DNA molecules in a nanopore catalyzed by phi29 DNA polymerase. J Am Chem Soc. 2010. 132(50): 17961-72. phi29 DNA polymerase is commercially available, see for example, New England Biolabs, Catalog No. M0269L.

As used herein, the term "Bst DNA polymerase" refers to a DNA polymerase with strand displacement activity obtained by cloning in *Bacillus stearothermophilus,* which possesses 5' to 3' DNA polymerase activity, but does not have 5' to 3' exonuclease activity and therefore does not have error correction function. Bst DNA polymerase is commercially available, see for example, Bst DNA polymerase series from New England Biolabs, for example, Bst1.0, Bst2.0, Bst3.0.

As used herein, the terms "loading", "immobilizing", and "attaching", when used in reference to nucleic acid, mean directly or indirectly attaching to a solid support via a covalent or noncovalent bond. In certain embodiments of the present disclosure, the method of the present invention comprises immobilizing a nucleic acid on a solid support via covalent attachment. Typically, however, it is only required that the nucleic acid remains immobilized or attached to the solid support under conditions where the use of the solid support is desired (e.g., in applications requiring nucleic acid amplification and/or sequencing). Alternatively, loading may occur by base pair hybridization or other means, such as covalent attachment as described above. Non-limiting examples of means of attachment of nucleic acids to solid supports include nucleic acid hybridization, biotin streptavidin binding, thiol binding, photoactivation binding, covalent binding, antibody-antigen, physical limitation via hydrogel or other porous polymer, etc. Various exemplary methods for immobilizing nucleic acids on solid supports can be found, for example, in G. Steinberg-Tatman et al., Bioconjugate Chemistry 2006, 17, 841-848; Xu X. et al. Journal of the American Chemical Society 128 ( 2006) 9286-9287; US patents or patent applications US 5639603, US 5641658, US2010248991; international patent applications WO 2001062982, WO 2001012862, WO 2007111937, WO0006770, and for all purposes, in particular for the preparation of solid supports with nucleic acids immobilized thereon, all related teachings are incorporated herein by reference in their entirety.

As used herein, the terms "chip" and "slide" have the same meaning and are used interchangeably.

As used herein, the term "primer" refers to a polynucleotide that can serve as a starting point for nucleic acid synthesis in a suitable buffer and at a suitable temperature under suitable conditions (i.e., in the presence of 4 different nucleoside triphosphates and reagents for polymerization, such as DNA or RNA polymerase or reverse transcriptase). Thus, the primer includes a target binding region that hybridizes to a target nucleic acid (template). Primers are generally oligonucleotides and are single-stranded. However, primers may refer to polynucleotides with double-stranded segments (e.g., partially double-stranded oligonucleotides). The appropriate length of the target binding region of the primer depends on the intended use of the primer. Short primer molecules generally require lower temperatures to form sufficiently stable hybrid complexes with the template. The primers need not reflect the exact sequence of the nucleic acid template, but must be sufficiently complementary to hybridize to the nucleic acid template.

In a first aspect, the present invention provides a method for loading a nucleic acid molecule on a solid support or for constructing a nucleic acid library immobilized on a solid support, comprising:
(1) providing a composition comprising a nucleic acid molecule, and the solid support;
(2) performing a first amplification of the nucleic acid molecule in the composition to obtain an amplification product comprising the nucleic acid molecule;
(3) suspending or slowing down the first amplification;
(4) loading the amplification product comprising the nucleic acid molecule onto the solid support; and
(5) performing a second amplification of the nucleic acid molecule in the amplification product loaded on the solid support.

In certain embodiments, the amplification product comprising the nucleic acid molecule is a nucleic acid nanoball.

In certain embodiments, the solid support has an array of sites for loading the amplification product (e.g., nucleic acid nanoball). In certain embodiments, the sites are arranged on the solid support in a predetermined pattern.

In certain embodiments, the nucleic acid molecule can be any type of nucleic acid. For example, the nucleic acid molecule can be deoxyribonucleic acid (DNA), ribonucleic acid (RNA), or an analog of DNA or RNA made from a nucleotide analog. The nucleic acid molecule can be single-stranded, double-stranded, or contain both single- and double-stranded sequences. The nucleic acid molecule can be derived from a double-stranded DNA (dsDNA) form (e.g., genomic DNA, PCR and amplification product, etc.), or can be derived from a single-stranded form such as DNA (ssDNA) or RNA. Furthermore, the nucleic acid molecule can be linear or circular. In certain embodiments, the nucleic acid molecule can be a single-stranded circularized nucleic acid molecule (e.g., single-stranded circularized DNA). In certain embodiments, the composition comprises a single-stranded circular nucleic acid molecule. In certain embodiments, the composition comprises a single-stranded circular DNA molecule.

In certain embodiments, the composition comprises one or more nucleic acid molecules, for example, at least 10, at least 20, at least 50, at least 100, at least 1000, at least 10⁴, at least 10⁵, at least 10⁶, at least 10⁷ or more nucleic acid molecules. In certain embodiments, the nucleic acid molecule is single-stranded circularized DNA. In certain embodiments, the nucleic acid molecule contains a nucleic acid fragment of interest of at least 100 bp, at least 200 bp, at least 500 bp, at least 800 bp, at least 1000 bp, at least 2000 bp in length.

In certain embodiments, in step (2), the size of the obtained amplification product (e.g., nucleic acid nanoball) does not exceed a predetermined size. In certain embodiments, in step (2), the obtained amplification product (e.g., nucleic acid nanoball) is a linear nucleic acid molecule, such as linear DNA.

In certain embodiments, the predetermined size is the maximum size of a nucleic acid molecule (e.g., nucleic acid nanoball) that can be accommodated by or attached by a single site on the solid support or the spacing of adjacent sites on the solid support.

In certain embodiments, the size of the obtained nucleic acid nanoball does not exceed 95%, 90%, 80%, 70%, 60%, 50%, 40%, 30%, 20% or 10% of the maximum size or the distance between the adjacent sites on the solid support.

In certain embodiments, in step (2), the first amplification of the nucleic acid molecule in the composition is performed by rolling circle amplification. In certain embodiments, the time of amplification (e.g., rolling circle amplification) is controlled so that the size of the amplification product (e.g., nucleic acid nanoball) does not exceed the predetermined size.

In certain embodiments, in step (3), the first amplification is suspended or slowed down by one or more methods selected from the group consisting of:
(a) controlling or reducing the amount of dNTPs in the reaction mixture for the first amplification; and/or
(b) inhibiting the activity of the nucleic acid polymerase used in the first amplification or inactivating the nucleic acid polymerase used in the first amplification.

In certain embodiments, the first amplification is suspended or slowed down by inhibiting the activity of the nucleic acid polymerase used in the first amplification or inactivating the nucleic acid polymerase used in the first amplification.

In certain embodiments, the activity of the nucleic acid polymerase used in the first amplification is inhibited so that the activity of the nucleic acid polymerase is reduced by at least 40%, at least 50%, at least 60%, at least 70%, at least 80%, at least 90%, or at least 95%.

In certain embodiments, the activity of the nucleic acid polymerase is inhibited or the nucleic acid polymerase is inactivated by one or more methods selected from the group consisting of:
(a) adding an activity inhibitor or denaturing agent of the nucleic acid polymerase;
(b) removing a cofactor required for the nucleic acid polymerase to function;
(c) adjusting the temperature of the reaction mixture for the first amplification to deviate from (e.g., above or below) the working temperature range of the nucleic acid polymerase;
(d) adjusting the pH of the reaction mixture for the first amplification to deviate from (e.g., above or below) the working pH range of the nucleic acid polymerase.

In certain embodiments, the nucleic acid polymerase is a DNA polymerase, for example, a DNA polymerase with strand displacement activity, for example, Bst DNA polymerase, phi29 DNA polymerase, and exo-Klenow.

It is easy to understand that the biological activity and stability of the enzyme (nucleic acid polymerases) are affected by many factors, such as temperature, pH, protein concentration, salt conditions, solvent or other factors. Those skilled in the art can change these factors according to actual needs, thereby affecting (e.g., reducing or restoring) the activity of the enzyme (nucleic acid polymerase). Thus, the methods of the present invention can affect enzymatic activity (e.g., reduce or restore enzymatic activity) by various suitable means and are not limited to the exemplary protocols or means described above. For example, the activity of a nucleic acid polymerase can be affected (e.g., reduced or restored) by changing the working conditions (e.g., working temperature and working pH) of the nucleic acid polymerase. Suitable working conditions for the nucleic acid polymerase can be readily determined by those skilled in the art. For example, suitable working conditions for phi29 DNA polymerase can be found in Nelson J.R. phi29 DNA polymerase-based methods for genomics applications [J]. Journal of Clinical Ligand Assay, 2002, 25(3): 276-279, which is incorporated herein in its entirety by reference.

In certain embodiments, the activity of the nucleic acid polymerase can be inhibited or the nucleic acid polymerase can be inactivated by removing a cofactor required for the nucleic acid polymerase to function. In certain embodiments, the cofactor is a cation (e.g., magnesium ion). In certain embodiments, the cofactor is removed by adding a cation chelating agent (e.g., a magnesium ion chelating agent), thereby inhibiting the activity of the nucleic acid polymerase or inactivating the nucleic acid polymerase. In certain embodiments, the magnesium ion chelating agent is selected from the group consisting of NTA, EDTA, HEDP, EDTMPS, DTPMPA, EDDHA, STPP, sodium dextrose and sodium metasilicate. In certain embodiments, the agent for chelating magnesium ion is EDTA.

In certain embodiments, the temperature of the reaction mixture for the first amplification is adjusted to deviates from (e.g., above or below) the working temperature range of the nucleic acid polymerase, thereby inhibiting the activity of the nucleic acid polymerase or inactivating the nucleic acid polymerase. In certain embodiments, the temperature of the reaction mixture is adjusted to be higher than the upper limit of the working temperature of the nucleic acid polymerase, for example, higher by at least 5°C, at least 10°C, at least 15°C, or more; alternatively, the temperature of the reaction mixture is adjusted to be lower than the lower limit of the working temperature of the nucleic acid polymerase, for example, lower by at least 5°C, at least 10°C, at least 15°C or more. In certain embodiments, the temperature of the reaction mixture is lowered to a temperature of ≤20°C, ≤10°C, ≤4°C, or lower; alternatively, the temperature of the reaction mixture is elevated to a temperature of ≥50°C, ≥ 60°C, ≥70°C, ≥80°C, or higher.

In certain embodiments, the pH of the reaction mixture for the first amplification can be adjusted by adding an acidic or basic buffer to deviate from (e.g., above or below) the working pH range of the nucleic acid polymerase, thereby inhibiting the activity of the nucleic acid polymerase or inactivating the nucleic acid polymerase. In certain embodiments, the pH of the reaction mixture is adjusted to be higher than the upper limit of the working pH of the nucleic acid polymerase, for example, higher by at least 1, at least 2, at least 3 or more pH units; or, the pH of the reaction mixture is adjusted to be lower than the lower limit of the working pH of the nucleic acid polymerase, for example, lower by at least 1, at least 2, at least 3 or more pH units. In certain embodiments, the acidic or basic buffer is selected from the group consisting of citrate buffer, phosphate buffer, acetate buffer, carbonate buffer and Tris hydrochloride buffer.

In certain embodiments, the activity of the nucleic acid polymerase can be inhibited or the nucleic acid polymerase can be inactivated by adding an activity inhibitor or denaturing agent of the nucleic acid polymerase. In certain embodiments, the activity inhibitor or denaturing agent of the nucleic acid polymerase is a surfactant, for example, selecting from the group consisting of sodium deoxycholate, sodium lauryl sarcosinate, sodium lauryl sulfate, Tween-20, NP-40 and Tritox-100.

In certain embodiments, the activity of the nucleic acid polymerase may be inhibited or the nucleic acid polymerase may be inactivated by one or more of the means described above.

In certain embodiments, the solid support is selected from the group consisting of latex bead, dextran bead, polystyrene surface, polypropylene surface, polyacrylamide gel, gold surface, glass surface, chip, sensor, electrode, and silicon wafer. In certain embodiments, the solid support is planar, spherical or porous. In certain embodiments, the maximum size of the nucleic acid molecule (e.g., nucleic acid nanoball) that can be accommodated by or attached to a single site on the solid support is ≤ 5000 nm, ≤ 2000 nm, ≤ 1000 nm, ≤ 700 nm, ≤ 500 nm, ≤ 300nm, or ≤100nm. In certain embodiments, the spacing of adjacent sites on the solid support is <5000 nm, < 2000 nm, < 1000 nm, < 700 nm, < 500 nm, < 300 nm, or < 100 nm.

In certain embodiments, in step (2), the first amplification of the nucleic acid molecules in the composition is performed by rolling circle amplification. In certain embodiments, in step (5), the second amplification of the nucleic acid molecule in the amplification product is performed by rolling circle amplification. In certain embodiments, the first amplification and the second amplification use the same or different (preferably the same) nucleic acid polymerase (e.g., DNA polymerase).

In certain embodiments, the solid support is pretreated prior to performing step (4); for example, by using poloxamer, enzyme (e.g., DNA polymerase (e.g., phi29 DNA polymerase), T4 ligation enzyme, BSA), or any combination thereof.

In certain embodiments, in step (5), the amplification product obtained by the second amplification is a linear nucleic acid molecule, such as linear DNA. In certain embodiments, the product obtained in step (5) is used as a nucleic acid sequencing library. In certain embodiments, after step (5), the nucleic acid molecule loaded on the solid support is sequenced.

In certain embodiments, in step (5), the second amplification of the nucleic acid molecule in the amplification product loaded on the solid support is performed by adding a reagent required for nucleic acid amplification. In certain embodiments, the reagent is selected from nucleic acid polymerase (e.g., DNA polymerase), dNTPs, working buffer for nucleic acid polymerase, and any combination thereof.

In certain embodiments, in step (5), the second amplification of the nucleic acid molecule in the amplification product loaded on the solid support is performed by eliminating a condition that causes the first amplification to be suspended or slowed down in step (3).

In certain embodiments, in step (3), the first amplification is suspended or slowed down by inhibiting the activity of the nucleic acid polymerase used for the first amplification, and, in step (5), the second amplification is performed by restoring the activity of the nucleic acid polymerase (e.g., by eliminating the condition that inhibits the activity of the nucleic acid polymerase). In certain embodiments, in step (5), the activity of the nucleic acid polymerase is restored to at least 60%, at least 70%, at least 80%, at least 90%, at least 95% or higher of its normal activity.

In certain embodiments, in step (3), the first amplification is suspended or slowed down by adding an activity inhibitor of the nucleic acid polymerase, and, in step (5), the activity of the nucleic acid polymerase is restored by removing the activity inhibitor, and the second amplification is performed.

In certain embodiments, in step (3), the first amplification is suspended or slowed down by removing a cofactor required for the nucleic acid polymerase to function, and, in step (5), the activity of the nucleic acid polymerase is restored by adding the cofactor, and the second amplification is performed.

In certain embodiments, in step (3), the first amplification is suspended or slowed down by adjusting the temperature of the reaction mixture to deviate from (e.g., above or below) the working temperature range of the nucleic acid polymerase, and, in step (5), the activity of nucleic acid polymerase is restored by adjusting the temperature of the reaction mixture to the working temperature range of the nucleic acid polymerase, and the second amplification is performed.

In certain embodiments, in step (3), the first amplification is suspended or slowed down by adjusting the pH of the reaction mixture to deviate from (e.g., above or below) the working pH range of the nucleic acid polymerase, and, in step (5), the nucleic acid polymerase activity is restored by adjusting the pH of the reaction mixture to the working pH range of the nucleic acid polymerase, and the second amplification is performed.

In certain embodiments, in step (3), the first amplification is suspended or slowed down by denaturing the nucleic acid polymerase, and, in step (5), the second amplification is performed by adding the nucleic acid polymerase.

In a second aspect, the present invention provides a kit, which comprises: a reagent for amplifying nucleic acid, a reagent for suspending or inhibiting nucleic acid amplification, and a reagent for loading the nucleic acid molecule onto a solid support. It is readily understood that the kit can be used to carry out the method of the present invention as described above. For example, the kit can be used to load a nucleic acid molecule on a solid support or to construct a nucleic acid library immobilized on a solid support.

In certain embodiments, the reagent for amplifying nucleic acid may comprise a nucleic acid polymerase, a working buffer (concentrated or non-concentrated) for the nucleic acid polymerase, a cofactor required for the nucleic acid polymerase to function, dNTPs, or any combination thereof. In certain embodiments, the nucleic acid polymerase is DNA polymerase, for example, DNA polymerase with strand displacement activity, for example, Bst DNA polymerase, phi29 DNA polymerase, and exo-Klenow. In certain embodiments, the cofactor is cation (e.g., magnesium ion). In certain embodiments, the working buffer is concentrated. For example, the working buffer can be concentrated at least 2-fold, at least 5-fold, or at least 10-fold.

In certain embodiments, the reagent for suspending or inhibiting nucleic acid amplification may comprise one or more selected from the group consisting of:
(1) an activity inhibitor or denaturing agent of the nucleic acid polymerase;
(2) a reagent capable of removing the cofactor required for the nucleic acid polymerase to function;
(3) an acidic or alkaline buffer capable of adjusting the pH of the solution.

In certain embodiments, the reagent for suspending or inhibiting nucleic acid amplification may comprises an activity inhibitor or denaturing agent of the nucleic acid polymerase. In certain embodiments, the activity inhibitor or denaturing agent of the nucleic acid polymerase is a surfactant, for example selected from the group consisting of sodium deoxycholate, sodium lauryl sarcosinate, sodium lauryl sulfate, Tween-20, NP-40 and Tritox-100.

In certain embodiments, the reagent for suspending or inhibiting nucleic acid amplification may comprise a reagent capable of removing the cofactor required for the nucleic acid polymerase to function. In certain embodiments, the cofactor is a cation (e.g., magnesium ion). In certain embodiments, the agent capable of removing the cofactor required for the nucleic acid polymerase to function is a cation chelating agent (e.g., magnesium ion chelating agent). In certain embodiments, the cation chelating agent (e.g., magnesium ion chelating agent) is selected from the group consisting of NTA, EDTA, HEDP, EDTMPS, DTPMPA, EDDHA, STPP, sodium dextrose, and sodium metasilicate. In certain embodiments, the cation chelating agent (e.g., magnesium ion chelating agent) is EDTA.

In certain embodiments, the reagent for suspending or inhibiting nucleic acid amplification may comprise an acidic or basic buffer capable of adjusting the pH of the solution. In certain embodiments, the acidic or basic buffer is selected from the group consisting of citrate buffer, phosphate buffer, acetate buffer, carbonate buffer and Tris hydrochloride buffer.

In certain embodiments, the reagent for loading the nucleic acid molecule onto the solid support comprises tripotassium citrate, citric acid, phi29 polymerase, Pluronic F68.

In certain embodiments, the kit further comprises a reagent for pretreating the solid support. In certain embodiments, the reagent for pretreating the solid support is selected from the group consisting of poloxamer, enzyme (e.g., DNA polymerase (e.g., phi29 DNA polymerase), T4 ligase, BSA), or any combination thereof.

### Beneficial effect

Compared with the prior art, the method for loading a nucleic acid nanoball (e.g., DNB) on a solid support provided by the present invention has higher loading density and loading stability. At the same time, the method of the present invention amplifies the target nucleic acid molecule in the nucleic acid nanoball (e.g., DNB) twice, and forms more copies of the target nucleic acid molecule on the slide, which can generate stronger and more stable signal, thereby facilitating the detection.

The method of the present invention can improve the quality of the DNB loaded on the high-density array, increase the sequencing target copy number of the array site, improve the signal-to-noise ratio of the array site, and allow longer read length and shorter imaging exposure time, as well as better overall data quality. This is particularly advantageous for high-throughput sequencing.

### Brief Description of the Drawings

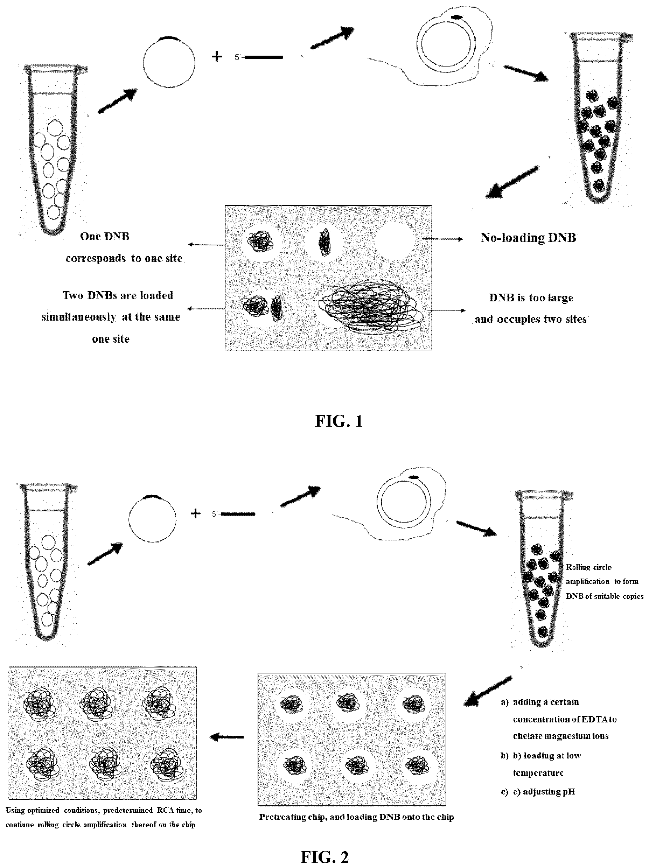

Fig. 1 shows the general procedure for loading DNB on a slide. In an exemplary embodiment, using a single-stranded circularized DNA library, DNB is obtained by performing rolling circle amplification with primer, dNTP and phi29 DNA polymerase or other polymerase with strand displacement activity under suitable conditions; after the rolling circle amplification is complete, the DNB is loaded directly onto a sequencing slide. Since each DNB may contain different number of copies of the nucleic acid molecule (some DNBs contain a higher number of copies and some DNBs contain a lower number of copies), the size of different DNB varies. During the loading process, due to the influence of Brown/thermal motion and shearing, etc., large DNBs may be loaded on two or more adjacent sites on the slide, while multiple small DNBs may be loaded at the same one site on the slide, and a few DNBs may even be lost into the solution, resulting in no-loading. This significantly affects the subsequent sequencing results (sequencing efficiency and sequencing quality).
Fig. 2 shows the procedure of loading a DNB onto a slide using the method of the present invention. In an exemplary embodiment, using a single-stranded circularized DNA library, a DNB is obtained by performing a first rolling circle amplification with primer, dNTP and phi29 DNA polymerase or other polymerase with strand displacement activity under suitable conditions. The conditions and time of the first rolling circle amplification are controlled to obtain a DNB of suitable size. In a preferred embodiment, the size of the DNB does not exceed the spacing of adjacent sites on the slide, whereby the DNB can be loaded onto the slide efficiently, avoiding simultaneous loading of multiple DNBs to the same site, or loading of one DNB to two or more adjacent sites on the slide. For example, when the site spacing of the chip is 700 nm, a rolling circle amplification time of no more than 20 min can be used; when the site spacing of the chip is 500 nm, a rolling circle amplification time of no more than 10 min can be used; when the site spacing is further reduced, less rolling circle amplification time can be used to obtain DNBs of suitable size.
   After a predetermined period of rolling circle amplification, various methods can be used to suspend or slow down the first rolling circle amplification. For example, methods that can be used include but are not limited to: (1) adding a certain concentration of EDTA to the reaction mixture to chelate magnesium ions, weaken or stop the polymerization ability of phi29 DNA polymerase, and stop or slow down the growth of DNB; (2) adjusting the temperature of the reaction mixture to a low temperature (e.g, below 20°C, such as 4°C) to weaken or stop the polymerization ability of phi29 DNA polymerase, and stop or slow down the growth of DNB; (3) adjusting the pH of the reaction mixture to acidity (e.g., using a pH=4.7 mixture of tripotassium citrate and citric acid) to weaken or stop the polymerization ability of phi29 DNA polymerase, and stop or slow down the growth of DNB.
   Subsequently, the DNB is loaded onto a sequencing slide, and a second rolling circle amplification is then performed on the slide to increase the number of copies of nucleic acid molecule contained in the DNB. Various methods can be used to perform the second rolling circle amplification. In certain embodiments, the rolling circle amplification can be restarted by eliminating conditions that inhibit phi29 DNA polymerase activity. For example, when EDTA is used in the previous step to chelate magnesium ions and weaken or stop the polymerization ability of phi29 DNA polymerase, after loading the DNB onto the sequencing slide, an appropriate concentration of magnesium ion buffer can be added to restore the polymerization ability of phi29 DNA polymerase, thereby continuing the rolling circle amplification on the slide. For example, when a low temperature (e.g., below 20°C, for example, 4°C) is used in the previous step to weaken or stop the polymerization ability of phi29 DNA polymerase, after the DNB is loaded onto the sequencing slide, the temperature of the reaction system can be elevated (e.g., 30-37°C) to restore the polymerization ability of phi29 DNA polymerase, thereby continuing the rolling circle amplification on the slide. For example, when an acidic buffer (e.g., a mixture of tripotassium citrate and citric acid at pH=4.7) is used to weaken or stop the polymerization ability of phi29 DNA polymerase in the previous step, after the DNB is loaded onto the sequencing slide, the pH of the reaction system can be elevated (for example, by using a working buffer of phi29 DNA polymerase or adding an alkaline buffer) to restore the polymerization ability of phi29 DNA polymerase, thereby continuing the rolling circle amplification on the slide.
   By using the above loading scheme of the present invention, the problem of low loading efficiency caused by the inappropriate size of the DNB used in the loading process (e.g., the size is too large) is effectively avoided, and it is effectively guaranteed that each site corresponds to one DNB; moreover, the number of copies of nucleic acid molecule contained in the DNB loaded at each site is higher, resulting in a stronger signal. Thus, the method of the present invention can improve the DNB loading efficiency and loading quality of each site on the chip (high-density array), improve the signal-to-noise ratio of each site, and allow longer read length and shorter imaging exposure time, and better overall data quality.
Fig. 3 shows the base signals of the first sequencing cycle of the experimental group 1 chip, the experimental group 2 chip and the control group chip.
Fig. 4 shows the DNB duplication rates (i.e., the probability that one DNB occupies two sites on the slide) at adjacent sites in the control group chip (Fig. 4A), the experimental group 1 chip (Fig. 4B), and the experimental group 2 chip (Fig. 4C).

### Specific Models for Carrying Out the Invention

The embodiments of the present invention will be described in detail below with reference to the examples, but those skilled in the art will understand that the following examples are only used to illustrate the present invention, and should not be regarded as limiting the scope of the present invention. If the specific conditions are not indicated in the examples, they are carried out according to the conventional conditions or the conditions suggested by the manufacturer. The reagents or instruments used without the manufacturer's indication are conventional products that can be obtained from the market.

The main equipment used in the examples of the present application were as follows: DNBSEQ-T7RS sequencer (purchased from MGI Co., Ltd. (MGI)), MGIDL-T7RS (purchased from MGI), DNBSEQ-T7RS chip (700 nm, purchased from MGI), Qubit^{®} 3.0 fluorescence quantitative instrument (purchased from Thermo), PCR machine (purchased from Trobot), highspeed centrifuge (purchased from Eppendorf).

The key reagents used in the examples of the present application were shown in Table 1 below:

**Table 1. Reagents used**

| Reagent Name | Brand |
|---|---|
| One strand sequencing primer | MGI |
| DNBSEQ-T7RS high-throughput sequencing kit | MGI |
| DNB SEQ-T7RS washing buffer kit | MGI |
| phi29 DNA polymerase | MGI |
| DNB rapid preparation kit | MGI |
| DNB SEQ-T7RS DNB rapid loading kit | MGI |
| 25mM dNTP Mix | 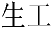 |
| 10%Pluronic F68 | Sigma |
| 1X TE buffer | MGI |
| Citric acid | Sigma |
| Tripotassium citrate | Sigma |
| 10X phi29 buffer | MGI |
| DNA nanoball loading buffer II | MGI |
| E. coli library | MGI |

### Example 1: Amplification of DNB and loading

In this example, experimental group 1, experimental group 2 and control group were set.
(1) The protocol of experimental group 1 was shown in Fig. 2, and the specific experimental process was as follows:

### 1.1 First amplification

According to the manufacturer's instructions, the MGIEasy^{™} DNA library preparation kit (MGI Co., Ltd.) was used to prepare the E. coli single-stranded circular DNA library with the main insert fragment at 300bp. The sequences of the two nucleic acid strands of the library adapter used were: 5'-AGT CGG AGG CCA AGC GGT CTT AGG AAG ACA ATC CTT TTG TAC AAC TCC TTG GCT CAC A-3' (SEQ ID NO: 1); and 5'-TTG TCT TCC TAA GGA ACG ACA TGG CTA CGA TCC GAC TT-3' (SEQ ID NO: 2). Then, according to the instructions of the DNB rapid preparation kit (MGI Co., Ltd.), 2 fmol/uL of single-stranded circular DNA library was taken, and then added with 20 uL of DNB primer mixture solution (the specific formula was shown in Table 2), wherein the RCA primer sequence was 5'-GCC ATG TCG TTC TGT GAG CCA AGG-3' (SEQ ID NO: 3). Primer hybridization was carried out on a PCR machine (95°C, 1 min → 65°C, 1 min → 40°C, 1 min → 4°C, 1 min); then 40uL of DNA polymerase mixture solution (specific formula was shown in Table 3) and 4ul of phi29 DNA polymerase (MGI Co., Ltd.) were added, and rolling circle amplification was carried out on a PCR machine (30°C, 20min). The prepared DNB was used for the subsequent loading onto DNBSEQ-T7 chip (the spacing of adjacent sites on the chip was 700 nm, and the maximum size of nucleic acid nanoball that could be accommodated by or attached to a single site was 220 nm).

**Table 2: DNB primer mixture solution**

| Reagent Name | Final concentration/Volume |
|---|---|
| 10X phi29 buffer | 1X |
| RCA primer | 1uM |
| H₂O | Supplemented to volume of 1ml |

**Table 3: DNB polymerase mixture solution**

| Reagent Name | Final concentration/Volume |
|---|---|
| 10X phi29 buffer | 1X |
| 25mM dNTP Mix | 400uM |
| 10%Pluronic F68 | 1% |
| 0.1U/ul Pyrophosphatase | 0.005U |
| H₂O | Supplemented to volume of 1ml |

After the rolling circle amplification was performed for the above time, the amplification reaction mixture was immediately placed on an ice box, added with 8 µL of 0.5mM EDTA, and slowly mixed with a wide-mouth pipette for 5-8 times, avoiding shaking and vigorous pipetting. Then, 2 µL of DNB was taken, and Qubit^{®} ssDNA Assay Kit (purchased from Thermo Fisher, art. No. Q10212) and Qubit^{®} 3.0 Fluorometer were used to perform concentration detection. The detection result showed that the nucleic acid concentration was 15ng/ul. Through 20min of rolling circle amplification, the size of the formed DNB was about 200nm. According to the loading volume of the DNBSEQ-T7 chip (MGI Co., Ltd.), a total of 5 tubes of DNB were prepared.

### 1.2 Preparing DNB loading system:

According to the instructions of DNBSEQ-T7RS DNB Rapid Loading Kit (MGI Co., Ltd.), 400ul of the DNB mixture prepared above was taken, added with 100ul of PBS, and slowly mixed with a wide-mouth pipette for 5-8 times. Centrifugation, shaking and vigorous pipetting should be avoided.

### 1.3 Pretreatment of sequencing slide and loading

First, slide pretreatment reagent 1 (the specific formula was shown in Table 4) was pumped into the chip, allowed to stand at room temperature for 5 minutes, and then slide pretreatment reagent 2 (the specific formula was shown in Table 5) was pumped therein, the two reagents were used for the pretreatment of the chip, which helped to assist in the efficient loading of DNB onto the sequencing slide. After the chip pretreatment was completed, the DNB loading reagent was loaded into MGIDL-T7RS equipment, therefore performing DNB loading. The loading was performed at 4°C for 30 min.

**Table 4: Slide pretreatment reagent 1**

| Reagent Name | Final concentration/Volume |
|---|---|
| 1M tripotassium citrate | 0.3M |
| 1M citric acid | 0.3M |
| 1X TE buffer | Supplemented to 1ml |
| 10U/ul phi29 polymerase | 10ul |

**Table 5: Slide pretreatment reagent 2**

| Reagent Name | Final concentration/Volume |
|---|---|
| 1M tripotassium citrate | 0.03M |
| 1M citric acid | 0.03M |
| 10%Pluronic F68 | 6ul |
| 1XTE buffer | Supplemented to 1ml |

### 1.4 Second amplification

After the loading was completed, 740ul of a mixture of magnesium ions with a final concentration of 5mM and DNB polymerase, and 1ul of a mixture of phi29 DNA polymerase were pumped therein to reactivate the polymerase, the temperature was set to 30°C, the second amplification was performed on the sequencing slide, and the amplification time was 30min. After the amplification was completed, the sequencing chip of experimental group 1 was obtained.

(2) The protocol of experimental group 2 was shown in Fig. 2, and the specific experimental process was as follows:

### 2.1 The first amplification

According to the manufacturer's instructions, the MGIEasy^{™} DNA library preparation kit (MGI Co., Ltd.) was used to prepare the E. coli single-stranded circular DNA library with the main insert fragment at 300bp. The sequences of the two nucleic acid strands of the library adapter used were: 5'-AGT CGG AGG CCA AGC GGT CTT AGG AAG ACA ATC CTT TTG TAC AAC TCC TTG GCT CAC A-3' (SEQ ID NO: 1); and 5'-TTG TCT TCC TAA GGA ACG ACA TGG CTA CGA TCC GAC TT-3' (SEQ ID NO: 2). Then, according to the instructions of the DNB rapid preparation kit (MGI Co., Ltd.), 2 fmol/uL of single-stranded circular DNA library was taken, and then added with 20 uL of DNB primer mixture solution (the specific formula was shown in Table 2), wherein the RCA primer sequence was 5'-GCC ATG TCG TTC TGT GAG CCA AGG-3' (SEQ ID NO: 3). Primer hybridization was carried out on a PCR machine (95°C, 1 min → 65°C, 1 min → 40°C, 1 min → 4°C, 1 min); then 40uL of DNA polymerase mixture solution (specific formula was shown in Table 3) and 4ul of phi29 DNA polymerase (MGI Co., Ltd.) were added, and rolling circle amplification was carried out on a PCR machine (30°C, 20min). The prepared DNB was used for the subsequent loading of DNBSEQ-T7 chip (the spacing of adjacent sites on the chip was 700 nm, and the maximum size of nucleic acid nanoball that could be accommodated by or attached to a single site was 220 nm).

**Table 2: DNB primer mixture solution**

| Reagent Name | Final concentration/Volume |
|---|---|
| 10X phi29 buffer | 1X |
| RCA primer | 1uM |
| H₂O | Supplemented to volume of 1ml |

**Table 3: DNB polymerase mixture solution**

| Reagent Name | Final concentration/Volume |
|---|---|
| 10X phi29 buffer | 1X |
| 25mM dNTP Mix | 400uM |
| 10%Pluronic F68 | 1% |
| 0.1U/ul Pyrophosphatase | 0.005U |
| H₂O | Supplemented to volume of 1ml |

After the rolling circle amplification was carried out for the above time, the amplification reaction mixture was immediately placed on an ice box, and gently mixed using a wide-mouth pipette for 5-8 times, avoiding shaking and vigorous pipetting. Then, 2 µL of DNB was taken, and Qubit^{®} ssDNA Assay Kit (purchased from Thermo Fisher, art. No. Q10212) and Qubit^{®} 3.0 Fluorometer were used for concentration detection. The detection result showed that the nucleic acid concentration was 15ng/ul. Through 20min of rolling circle amplification, the size of the formed DNB was about 200nm. According to the loading volume of the DNBSEQ-T7 chip (MGI Co., Ltd.), a total of 5 tubes of DNB were prepared.

### 2.2 Preparation of DNB loading system:

According to the instructions of the DNBSEQ-T7RS DNB Rapid Loading Kit (MGI Co., Ltd.), 500ul of the DNB mixture prepared above was taken and placed in a loading DNB tube, and immediately placed on ice for later use.

### 2.3 Pretreatment of sequencing slide and loading

First, slide pretreatment reagent 1 (the specific formula was shown in Table 4) was pumped in the slide, allowed to stand at room temperature for 5 minutes, and then slide pretreatment reagent 2 (the specific formula was shown in Table 5) was pumped therein, and the two reagents were used to perform the pretreatment of the chip, which helped to assist in the efficient loading of DNB onto the sequencing slide. After the chip pretreatment was completed, the MGIDL-T7RS equipment was used to load the DNB loading system, so as to perform DNB loading. The loading was performed at 4°C for 30 min.

**Table 4: Slide pretreatment reagent 1**

| Reagent Name | Final concentration/Volume |
|---|---|
| 1M tripotassium citrate | 0.3M |
| 1M citric acid | 0.3M |
| 1X TE buffer | Supplemented to 1ml |
| 10U/ul phi29 polymerase | 10ul |

**Table 5: Slide pretreatment reagent 2**

| Reagent Name | Final concentration/Volume |
|---|---|
| 1M tripotassium citrate | 0.03M |
| 1M citric acid | 0.03M |
| 10%Pluronic F68 | 6ul |
| 1XTE buffer | Supplemented to 1ml |

### 2.4 Second amplification

After the loading was performed at 4 °C for 30 min, the temperature was set to 30°C, so as to reactivate the phi29 enzyme, the second amplification was performed on the sequencing slide with an amplification time of 30 min. After the amplification was completed, the sequencing chip of experimental group 2 was obtained.

(3) The protocol of the control group was shown in Fig. 1, and the specific experimental process was as follows:
The sample used in the control group was the same as that in the experimental group, that was, the E. coli single-stranded circular DNA library with the insert fragment mainly at 300 bp. The sample was subjected to rolling circle amplification using the same kit and the same protocol as the experimental group. Due to the requirements of the sequencing protocol on copy number and sequencing signal, the amplification time in the control group was set to 40 min. The nucleic acid concentration of the amplification product was 35 ng/ul, and the average size of the formed DNB was about 300 nm.

Subsequently, using the same kit and the same protocol as the experimental group, the amplification product (DNB) was loaded onto the sequencing slide, and the second rolling circle amplification was not performed (i.e., step 1.4 was not performed), thereby obtaining the sequencing chip of control group.

### Example 2: Sequencing

According to the instructions of DNBSEQ-T7RS high-throughput sequencing kit (MGI Co., Ltd.) and DNBSEQ-T7RS washing buffer kit (MGI Co., Ltd.), the sequencing chips obtained in the above experimental group 1, experimental group 2 and control group were placed on the DNBSEQ-T7RS sequencer, and PE100 (paired-end 100bp) sequencing was performed respectively. The sequence of the one-strand sequencing primer (MGI Co., Ltd.) used in the sequencing process was 5'-GC TCA CAG AAC GAC ATG GCT ACG ATC CGA CTT-3' (SEQ ID NO: 4).

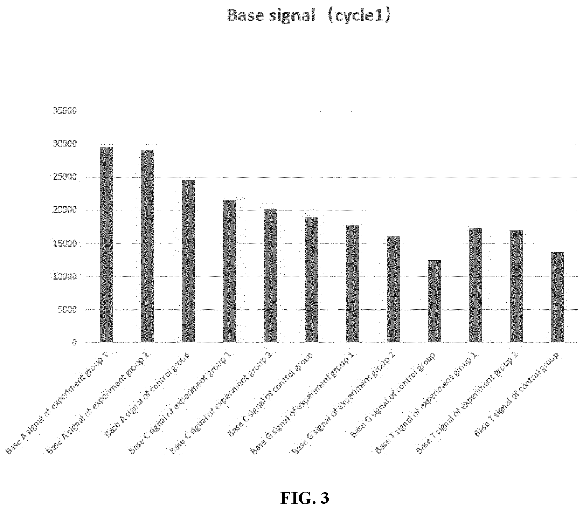

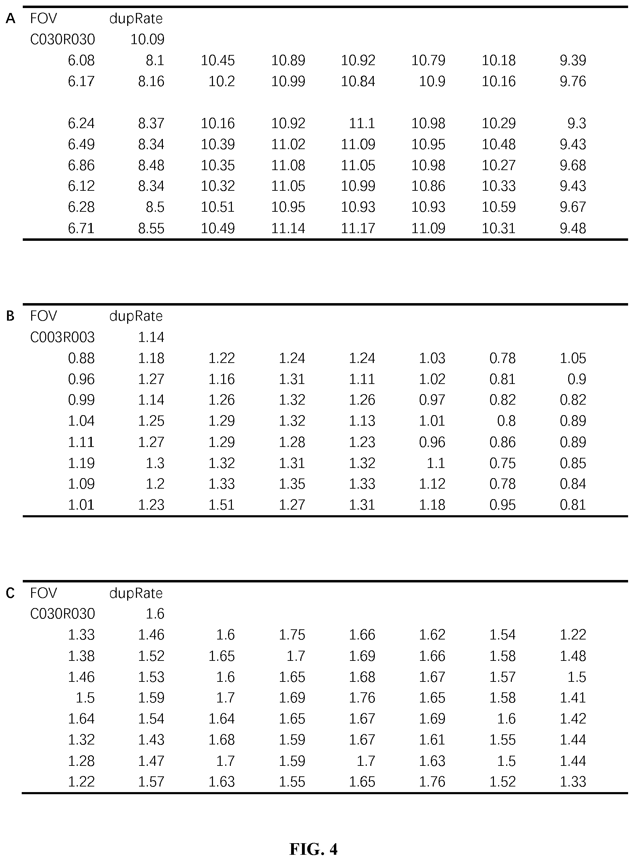

Fig. 3 shows the comparison results of the base signals of the experimental group 1 chip, the experimental group 2 chip and the control group chip in the first sequencing cycle. The results showed that the signals of the experimental group 1 and experimental group 2 were higher than those of the control group. Table 6 shows the PE100 sequencing report in the experimental group 1, the experimental group 2 and the control group of the present invention. The results showed that the total data volume (total reads) and sequencing quality (Q30) of the experimental group 1 and experimental group 2 using the protocol of the present invention were better than those of the control group. Fig. 4 shows the values of the duplication rate of adjacent DNBs in the experimental group 1, experimental group 2 and control group of the present invention. The higher the value, the more serious the phenomenon that one DNB occupied two site positions. It could be seen from Fig. 4, the duplication rate (dup-rate) of the control group (Fig. 4A) was 10.09%, while the duplication rate (dup-rate) of the experimental group 1 (Fig. 4B) was 1.14%, and the duplication rate (dup-rate) of the experimental group 2 (Fig. 4C) was 1.6%. The results showed that the duplication rates of the experimental groups according to the protocol of the present invention were much lower than that of the control group.

**Table 6. PE100 sequencing data of experimental groups and control group**

| | Control group | Experimental group 1 | Experimental group 2 |
|---|---|---|---|
| Sequencing cycles | 210 | 210 | 210 |
| Total reads (M) | 4813 | 5355 | 5200 |
| Q30 | 87.35 | 90.72 | 90.5 |
| Resolution ratio (%) | 99.01 | 99.01 | 99.00 |

Although specific embodiments of the present invention have been described in detail, those skilled in the art will understand that various modifications and changes can be made to the details in light of all the teachings that have been disclosed, and that these changes are all within the scope of the present invention. The full scope of the invention is given by the appended claims and any equivalents thereof.

## Claims

1. A method for loading a nucleic acid molecule on a solid support or for constructing a nucleic acid library that is immobilized on a solid support, comprising:
(1) providing a composition comprising a nucleic acid molecule, and the solid support;
(2) performing a first amplification of the nucleic acid molecule in the composition to obtain an amplification product comprising the nucleic acid molecule;
(3) suspending or slowing down the first amplification;
(4) loading the amplification product comprising the nucleic acid molecule onto the solid support; and
(5) performing a second amplification of the nucleic acid molecule in the amplification product loaded on the solid support;
preferably, the amplification product comprising the nucleic acid molecule is a nucleic acid nanoball;
preferably, the solid support has an array of sites for loading the amplification product (e.g., nucleic acid nanoball);
preferably, the sites are arranged on the solid support in a predetermined pattern;
preferably, the nucleic acid molecule is selected from the group consisting of deoxyribonucleic acid (DNA), ribonucleic acid (RNA), or an analog of DNA or RNA;
preferably, the composition comprises a single-stranded circular nucleic acid molecule; preferably, the composition comprises a single-stranded circular DNA molecule;
preferably, the composition comprises one or more nucleic acid molecules, such as at least 10, at least 20, at least 50, at least 100, at least 1000, at least 10⁴, at least 10⁵, at least 10⁶, at least 10⁷ or more nucleic acid molecules; preferably, the nucleic acid molecules are single-stranded circularized DNAs;
preferably, the nucleic acid molecule comprises a target nucleic acid fragment with a length of at least 100 bp, at least 200 bp, at least 500 bp, at least 800 bp, at least 1000 bp, or at least 2000 bp.

2. The method according to claim 1, wherein, in step (2), the size of the obtained amplification product (e.g., nucleic acid nanoball) does not exceed a predetermined size;
preferably, in step (2), the obtained amplification product (e.g., nucleic acid nanoball) is a linear nucleic acid molecule, such as linear DNA;
preferably, the predetermined size is the maximum size of the nucleic acid molecule (e.g., nucleic acid nanoball) that can be accommodated by or attached to a single site in the solid support or is the spacing of adjacent sites in the solid support;
preferably, the size of the obtained nucleic acid nanoball does not exceed 95%, 90%, 80%, 70%, 60%, 50%, 40%, 30%, 20% or 10% of the maximum size or the spacing of adjacent sites;
preferably, in step (2), the first amplification of the nucleic acid molecule in the composition carried out by rolling circle amplification;
preferably, by controlling the time of amplification (e.g., rolling circle amplification), the size of the amplification product (e.g., nucleic acid nanoball) does not exceed the predetermined size.

3. The method according to claim 1 or 2, wherein, in step (3), the first amplification is suspended or slowed down by one or more methods selected from the group consisting of:
(a) controlling or reducing the amount of dNTPs in the reaction mixture for the first amplification; and/or
(b) inhibiting the activity of the nucleic acid polymerase used in the first amplification or inactivating the nucleic acid polymerase used in the first amplification;
preferably, the first amplification is suspended or slowed down by inhibiting the activity of the nucleic acid polymerase used in the first amplification or inactivating the nucleic acid polymerase used in the first amplification;
preferably, the activity of the nucleic acid polymerase used in the first amplification is inhibited, so that the activity of the nucleic acid polymerase is reduced by at least 40%, at least 50%, at least 60%, at least 70%, at least 80%, at least 90%, or at least 95%;
preferably, the activity of the nucleic acid polymerase is inhibited or the nucleic acid polymerase is inactivated by one or more methods selected from the group consisting of:
(a) adding an activity inhibitor or denaturing agent of the nucleic acid polymerase;
(b) removing a cofactor required for the nucleic acid polymerase to function;
(c) adjusting the temperature of the reaction mixture for the first amplification to deviate from (e.g., above or below) the working temperature range of the nucleic acid polymerase;
(d) adjusting the pH of the reaction mixture for the first amplification to deviate from (e.g., above or below) the working pH range of the nucleic acid polymerase;
preferably, the nucleic acid polymerase is a DNA polymerase, for example a DNA polymerase with strand displacement activity, such as Bst DNA polymerase, phi29 DNA polymerase and exo-Klenow.

4. The method according to claim 3, wherein the activity of the nucleic acid polymerase is inhibited or the nucleic acid polymerase is inactivated by removing a cofactor required for the nucleic acid polymerase to function;
preferably, the cofactor is a cation (e.g., magnesium ion);
preferably, the cofactor is removed by adding a cation chelating agent (e.g., a magnesium ion chelating agent), thereby inhibiting the activity of the nucleic acid polymerase or inactivating the nucleic acid polymerase;
preferably, the magnesium ion chelating agent is selected from the group consisting of NTA, EDTA, HEDP, EDTMPS, DTPMPA, EDDHA, STPP, sodium dextrose and sodium metasilicate;
preferably, the reagent for chelating magnesium ion is EDTA.

5. The method according to claim 3, wherein the temperature of the reaction mixture for the first amplification is adjusted to deviate from (e.g., above or below) the working temperature range of the nucleic acid polymerase, thereby inhibiting the activity of the nucleic acid polymerase or inactivating the nucleic acid polymerase;
preferably, the temperature of the reaction mixture is adjusted to be higher than the upper limit of the working temperature of the nucleic acid polymerase, for example, higher by at least 5°C, at least 10°C, at least 15°C or more; or, the temperature of the reaction mixture is adjusted to be lower than the lower limit of the working temperature of the nucleic acid polymerase, for example, lower by at least 5°C, at least 10°C, at least 15°C or more;
preferably, the temperature of the reaction mixture is lowered to a temperature of ≤20°C, ≤10°C, ≤4°C, or lower; or, the temperature of the reaction mixture is elevated to a temperature of ≥50°C, ≥60°C, ≥ 70°C, ≥80°C, or higher.

6. The method according to claim 3, wherein the pH of the reaction mixture for the first amplification is adjusted by adding an acidic or basic buffer to deviate from (e.g., above or below) the working pH range of the nucleic acid polymerase, thereby inhibiting the activity of the nucleic acid polymerase or inactivating the nucleic acid polymerase;
preferably, the pH of the reaction mixture is adjusted to be higher than the upper limit of the working pH range of the nucleic acid polymerase, for example higher by at least 1, at least 2, at least 3 or more pH units; or, the pH of the reaction mixture is adjusted to be lower than the lower limit of the working pH range of the nucleic acid polymerase, for example, lower by at least 1, at least 2, at least 3 or more pH units;
preferably, the acidic or basic buffer is selected from the group consisting of citrate buffer, phosphate buffer, acetate buffer, carbonate buffer and Tris hydrochloride buffer.

7. The method according to claim 3, wherein the activity of the nucleic acid polymerase is inhibited or the nucleic acid polymerase is inactivated by adding an activity inhibitor or denaturing agent of the nucleic acid polymerase; preferably, the activity inhibitor or denaturing agent of the nucleic acid polymerase is a surfactant, for example, selected from the group consisting of sodium deoxycholate, sodium lauryl sarcosinate, sodium lauryl sulfate, Tween-20, NP-40 and Tritox-100.

8. The method according to any one of claims 1-7, wherein the method has one or more features selected from the group consisting of:
(a) the solid support is selected from the group consisting of latex bead, dextran bead, polystyrene surface, polypropylene surface, polyacrylamide gel, gold surface, glass surface, chip, sensor, electrode and silicon wafer;
(b) the solid support is planar, spherical or porous;
(c) the maximum size of the nucleic acid molecule (e.g., nucleic acid nanoball) that can be accommodated by or attached to a single site in the solid support is ≤ 5000 nm, ≤ 2000 nm, ≤ 1000 nm, ≤ 700 nm, ≤ 500 nm, ≤ 300 nm, or ≤ 100nm;
(d) in step (2), the first amplification of the nucleic acid molecule in the composition is carried out by rolling circle amplification;
(e) in step (5), the second amplification of the nucleic acid molecule in the amplification product is carried out by rolling circle amplification;
(f) the first amplification and the second amplification use the same or different (preferably the same) nucleic acid polymerase (e.g., DNA polymerase);
(g) the solid support is subjected to pretreatment prior to performing step (4); for example, the solid support is pretreated by using poloxamer, enzyme (e.g., DNA polymerase (e.g., phi29 DNA polymerase), T4 ligase, BSA), or any combination thereof; and
(h) in step (5), the amplification product obtained by the second amplification is a linear nucleic acid molecule, such as linear DNA;
(i) the product obtained in step (5) is used as a nucleic acid sequencing library;
(j) the method further comprises, after step (5), sequencing the nucleic acid molecule loaded on the solid support.

9. The method according to any one of claims 1-8, wherein, in step (5), the second amplification of the nucleic acid molecules in the amplification product loaded on the solid support is performed by adding a reagent required for nucleic acid amplification;
preferably, in step (5), the activity of the nucleic acid polymerase is restored to at least 60%, at least 70%, at least 80%, at least 90%, at least 95% or higher of its normal activity;
preferably, the reagent is selected from the group consisting of nucleic acid polymerase (e.g., DNA polymerase), dNTPs, working buffer for nucleic acid polymerase, and any combination thereof.

10. The method according to any one of claims 1-8, wherein, in step (5), by eliminating a condition that causes the first amplification to be suspended or slowed down in step (3), the second amplification of the nucleic acid molecule in the amplification product loaded on the solid support is performed;
preferably, in step (3), the first amplification is suspended or slowed down by inhibiting the activity of the nucleic acid polymerase used for the first amplification, and, in step (5), the second amplification is performed by restoring the activity of the nucleic acid polymerase (e.g., by eliminating a condition that inhibits the activity of the nucleic acid polymerase);
for example, in step (3), the first amplification is suspended or slowed down by adding an activity inhibitor of the nucleic acid polymerase, and, in step (5), the activity inhibitor is removed to restore the activity of the nucleic acid polymerase, and the second amplification is performed;
for example, in step (3), the first amplification is suspended or slowed down by removing a cofactor required for the nucleic acid polymerase to function, and, in step (5), the activity of the nucleic acid polymerase is restored by adding the cofactor, and the second amplification is performed;
for example, in step (3), the first amplification is suspended or slowed down by adjusting the temperature of the reaction mixture to deviate from (e.g., above or below) the working temperature range of the nucleic acid polymerase, and, in step (5), the activity of the nucleic acid polymerase is restored by adjusting the temperature of the reaction mixture to the working temperature range of the nucleic acid polymerase, and the second amplification is performed;
for example, in step (3), the first amplification is suspended or slowed down by adjusting the pH of the reaction mixture to deviate from (e.g., above or below) the working pH range of the nucleic acid polymerase, and, in step (5), the activity of the nucleic acid polymerase is restored by adjusting the pH of the reaction mixture to the working pH range of the nucleic acid polymerase, and the second amplification is performed;
for example, in step (3), the first amplification is suspended or slowed down by denaturing the nucleic acid polymerase, and, in step (5), the second amplification is performed by adding the nucleic acid polymerase.

11. A kit, which comprises: a reagent for amplifying a nucleic acid, a reagent for suspending or inhibiting nucleic acid amplification, and a reagent for loading the nucleic acid molecule onto a solid support;
preferably, the reagent for amplifying nucleic acid comprises a nucleic acid polymerase, a working buffer (concentrated or non-concentrated) for the nucleic acid polymerase, a cofactor required for the nucleic acid polymerase to function, dNTPs, or any combination thereof;
preferably, the nucleic acid polymerase is a DNA polymerase, for example a DNA polymerase with strand displacement activity, such as Bst DNA polymerase, phi29 DNA polymerase and exo-Klenow;
preferably, the cofactor is a cation (e.g., magnesium ion);
preferably, the working buffer is concentrated; for example, the working buffer is concentrated at least 2-fold, at least 5-fold or at least 10-fold;
preferably, the reagent for suspending or inhibiting nucleic acid amplification may comprise one or more selected from the group consisting of:
(1) an activity inhibitor or denaturing agent of the nucleic acid polymerase;
(2) a reagent capable of removing a cofactor required for the nucleic acid polymerase to function;
(3) an acidic or alkaline buffer capable of adjusting the pH of solution;
preferably, the agent for suspending or inhibiting nucleic acid amplification may comprise an activity inhibitor or denaturing agent of the nucleic acid polymerase;
preferably, the activity inhibitor or denaturing agent of the nucleic acid polymerase is a surfactant, for example, selected from the group consisting of sodium deoxycholate, sodium lauryl sarcosinate, sodium lauryl sulfate, Tween-20, NP-40 and Tritox-100;
preferably, the reagent for suspending or inhibiting nucleic acid amplification comprises a reagent capable of removing a cofactor required for the nucleic acid polymerase to function;
preferably, the cofactor is a cation (e.g., magnesium ion);
preferably, the reagent capable of removing the cofactor required for nucleic acid polymerase to function is a cation chelating agent (e.g., a magnesium ion chelating agent);
preferably, the cation chelating agent (e.g., magnesium ion chelating agent) is selected from the group consisting of NTA, EDTA, HEDP, EDTMPS, DTPMPA, EDDHA, STPP, sodium dextrose and sodium metasilicate;
preferably, the cation chelating agent (e.g., magnesium ion chelating agent) is EDTA;
preferably, the reagent for suspending or inhibiting nucleic acid amplification comprises an acidic or basic buffer capable of adjusting the pH of solution;
preferably, the acidic or basic buffer is selected from the group consisting of citrate buffer, phosphate buffer, acetate buffer, carbonate buffer and Tris hydrochloric acid buffer;
preferably, the reagent for loading the nucleic acid molecule onto the solid support comprises tripotassium citrate, citric acid, phi29 polymerase, Pluronic F68;
preferably, the kit further comprises a reagent for pretreating the solid support;
preferably, the reagent for pretreating the solid support is selected from the group consisting of poloxamer, enzyme (e.g., DNA polymerase (e.g., phi29 DNA polymerase), T4 ligase, BSA), or any combination thereof.
